(19) Europäisches Patentamt
European Patent Office
Office européen des brevets

(11) Veröffentlichungsnummer: **0 183 147**
**A2**

# (12) EUROPÄISCHE PATENTANMELDUNG

(21) Anmeldenummer: **85114562.3**

(22) Anmeldetag: **16.11.85**

(51) Int. Cl.[4]: **C 07 D 249/08**
C 07 D 233/61, A 01 N 43/653
A 01 N 43/50

(30) Priorität: **30.11.84 JP 251981/84**

(43) Veröffentlichungstag der Anmeldung:
**04.06.86 Patentblatt 86/23**

(84) Benannte Vertragsstaaten:
**AT BE CH DE FR GB IT LI NL**

(71) Anmelder: **NIHON TOKUSHU NOYAKU SEIZO K.K.**
**No.4, 2-chome, Nihonbashi Honcho Chuo-ku**
**Tokyo 103(JP)**

(72) Erfinder: **Kurahashi, Yoshio**
**47-15, Oya-machi**
**Hachioji-shi Tokyo(JP)**

(72) Erfinder: **Morishima, Norihisa**
**4-32-5, Owada-cho**
**Hachioji-shi Tokyo(JP)**

(72) Erfinder: **Yamaguchi, Naoko**
**4-6-7, Shinmei**
**Hino-shi Tokyo(JP)**

(72) Erfinder: **Matsumoto, Noboru**
**39-15, Namiki-cho**
**Hachioji-shi Tokyo(JP)**

(74) Vertreter: **Schumacher, Günter, Dr. et al,**
**c/o Bayer AG Konzernverwaltung RP Patentabteilung**
**D-5090 Leverkusen 1 Bayerwerk(DE)**

## (54) beta-Substituierte Aminophenethylazol-Derivate.

(57) Neue β-substituierte Aminophenethylazol-Derivate der Formel (I)

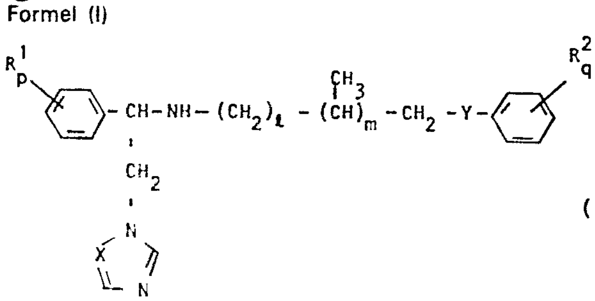

(I)

in welcher
X, Y, $R^1$, $R^2$, l, m, p und q die in der Beschreibung angegebene Bedeutung haben,
ein Verfahren zur Herstellung der neuen Stoffe und deren Verwendung als Fungizide.

Neue Zwischenprodukte der Formel (IV)

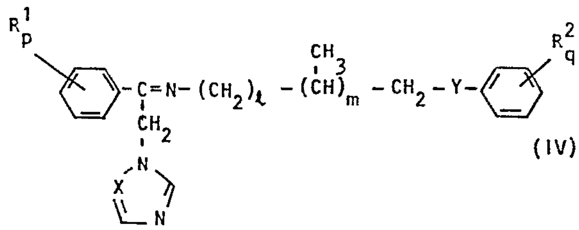

(IV)

in welcher
X, Y, $R^1$, $R^2$, l, m, p und q die in der Beschreibung angegebene Bedeutung haben,
und ein Verfahren zur Herstellung der neuen Zwischenprodukte.

 

## ß-Substituierte Aminophenethylazol-Derivate

Die vorliegende Erfindung betrifft neue ß-substituierte Aminophenethylazol-Derivate, ein Verfahren zu ihrer Herstellung, ihre Verwendung als Fungizide für Landwirtschaft und Gartenbau sowie neue Zwischenprodukte für ihre Herstellung und ein Verfahren zu deren Herstellung.

Es wurde bereits offenbart, daß bestimmte 1-Phenethylimidazol-Derivate antifungale und antibakterielle Wirksamkeit besitzen und gegen Verursacher menschlicher und tierischer Krankheiten sowie gegen Pilzbefall in der Landwirtschaft wirksam sind (vgl. die JP-OS 49369/1981).

Nunmehr wurden neue ß-substituierte Aminophenethylazol-Derivate der Formel (I)

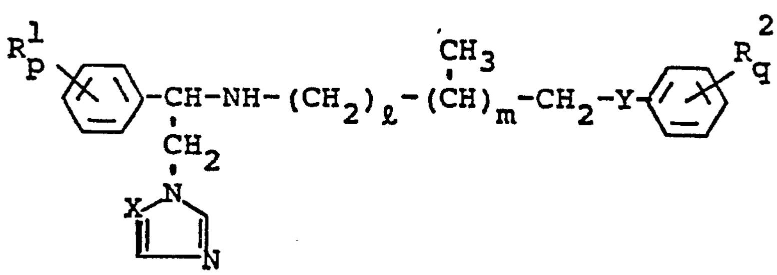 (I)

,

gefunden, in der

X N oder CH bezeichnet,

Y ein Sauerstoff- oder Schwefel-Atom bezeichnet,

$R^1$ eine Niederalkyl-Gruppe, ein Halogen-Atom oder eine Niederalkoxy-Gruppe bezeichnet,

$R^2$ eine Niederalkyl-Gruppe, ein Halogen-Atom, eine Niederalkoxy-Gruppe, eine Trifluoromethyl-Gruppe, eine Nitro-Gruppe oder eine Methoxyiminomethyl-Gruppe bezeichnet,

p 0, 1 oder 2 bezeichnet,

q 0, 1, 2 oder 3 bezeichnet,

$\ell$ 1, 2, 3, 4 oder 5 bezeichnet und

m 0 oder 1 bezeichnet.

ß-substituierte Aminophenethylazol-Derivate der Formel (I) werden erhalten, indem man Verbindungen der Formel (IV)

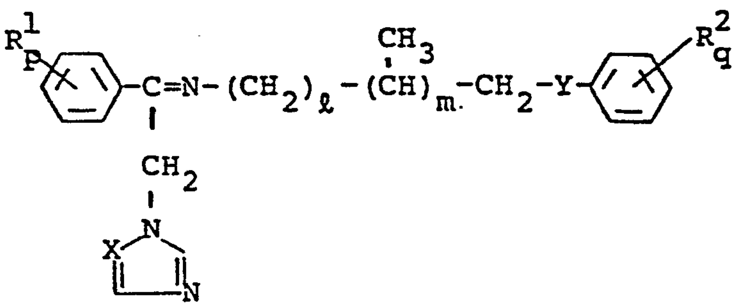

(IV)

in welcher

X, Y, $R^1$, $R^2$, p, q, l und m die oben angebene Bedeutung haben,

gegebenenfalls in Gegenwart eines inerten Verdünnungsmittels reduziert.

Verbindungen der Formel (IV) lassen sich herstellen, indem man Verbindungen der Formel (II)

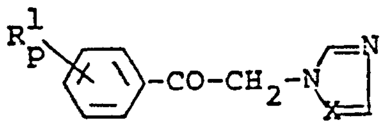

(II) ,

in welcher

X, $R^1$ und p die oben angegebene Bedeutung haben,

mit Verbindungen der Formel (III)

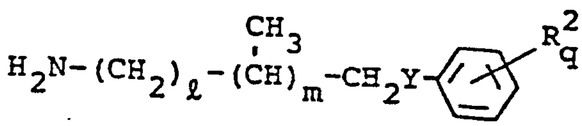

(III) ,

in welcher

Y, $R^2$, q, l und m die oben angegebene Bedeutung haben,

gegebenenfalls in Gegenwart eines inerten Verdünnungsmittels umsetzt.

Die neuen ß-substituierten Aminophenylethylazol-Derivate der Formel (I) haben hochwirksame, in Landwirtschaft und Gartenbau nutzbare Eigenschaften.

Weiterhin gefunden wurden neue Verbindungen der oben angegebenen Formel (IV) und deren Verwendung als Zwischenprodukte für die Herstellung von Verbindungen der Formel (I).

Überraschenderweise zeigen die erfindungsgemäßen ß-substituierten Aminophenethylazol-Derivate der Formel (I) beim Einsatz auf den Gebieten von Landwirtschaft und Gartenbau eine wesentlich stärkere fungizide Wirkung als die aus dem Stand der Technik bekannten, konstitutionell ähnlichsten Verbindungen, beispielsweise aus dem oben angeführten Stand der Technik. Im einzelnen ist diese überraschende Wirkung am stärksten bemerkenswert gegen die Graufäule (Botrytis cinerea).

Unter den ß-substituierten Aminophenethylazol-Derivaten der Formel (I) sind diejenigen bevorzugt, in denen

X N bezeichnet,

Y ein Sauerstoff- oder Schwefel-Atom bezeichnet,

$R^1$ eine Alkyl-Gruppe mit 1 bis 4 Kohlenstoff-Atomen ausgewählt aus Methyl, Ethyl, n-(oder iso-)Propyl und n-(oder iso-, sec- und tert-)Butyl, ein Halogen-Atom, ausgewählt aus Fluor, Chlor, Brom und Iod, oder eine Alkoxy-Gruppe mit einer der oben beispielhaft genannten $C_{1-4}$-Alkyl-Gruppen bezeichnet,

$R^2$ eine Alkyl-Gruppe mit 1 bis 4 Kohlenstoff-Atomen wie oben beispielhaft genannt, ein Halogen-Atom, eine Alkoxy-Gruppe mit 1 bis 4 Kohlenstoff-Atomen, eine Trifluormethyl-Gruppe, eine Nitro-Gruppe oder eine Methoximinomethyl-Gruppe bezeichnet,

p 0, 1 oder 2 bezeichnet,
q 0, 1, 2 oder 3 bezeichnet,
ℓ 1, 2 oder 3 bezeichnet und
m 0 oder 1 bezeichnet.

In besonderer Weise bevorzugte ß-substituierte Aminophenethylazol-Derivate der Formel (I) sind diejenigen, in denen

X N bezeichnet,
Y ein Sauerstoff-Atom bezeichnet,
$R^1$ eine Methyl-Gruppe oder ein Chlor-Atom bezeichnet,
$R^2$ eine Methyl-Gruppe, ein Fluor-Atom, ein Chlor-Atom, eine Methoxy-Gruppe, eine Trifluormethyl-Gruppe oder eine Nitro-Gruppe bezeichnet,
p 2 bezeichnet,
q 0 oder 1 bezeichnet,
l 1 oder 2 bezeichnet und
m 0 oder 1 bezeichnet.

Speziell erwähnt seien die folgenden Verbindungen:
1-/ß-(2-Phenoxyethylamino)-2,4-dichlorphenethyl7-1H-1,2,4-triazol,
1-/ß-(3-Phenoxypropylamino)-2,4-dichlorphenethyl7-1H-1,2,4-triazol,
1-/ß-/3-(4-chlorphenoxy)-propylamino7-2,4-dichlorphenethyl7-1H-1,2,4-triazol,
1-/ß-(2-Methyl-3-phenoxypropylamino)-2,4-dichlorphenethyl7-1H-1,2,4-triazol und
1-/ß-/3-(4-tolyloxy)propylamino7-2,4-dichlorphenethyl7-1H-1,2,4-triazol.

Verwendet man 1-[β-(3-Phenoxypropylimino)-2,4-dichlor-phenethyl]-1H-1,2,4-triazol als Ausgangsstoff und Natriumbohydrid als Reduktionsmittel, so kann der Verlauf des erfindungsgemäßen Verfahrens durch das folgende Reaktionsschema veranschaulicht werden:

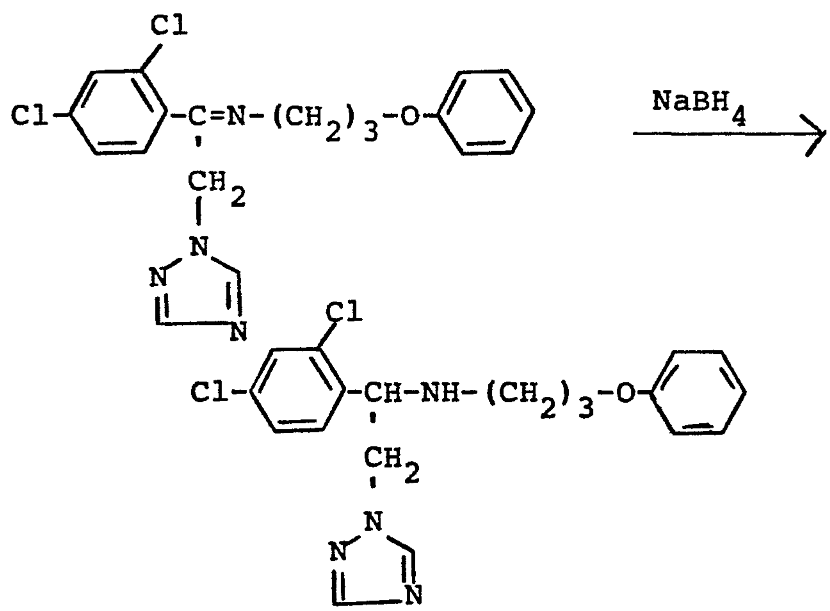


Verwendet man 1-(2,4-Dichlorbenzoylmethyl)-1,2,4-triazol und 3-Phenoxypropylamin als Ausgangsstoffe, so kann der Verlauf des Verfahren zur Herstellung von Zwischenprodukten der Formel (IV) durch das folgende Formelschema veranschaulicht werden:

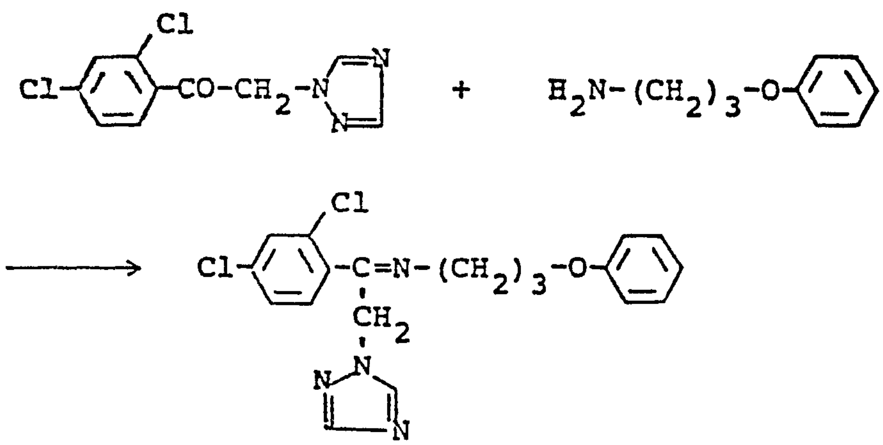

Die bei der Herstellung von Zwischenprodukten der Formel (IV) als Ausgangsstoffe benötigten Verbindungen sind durch die Formel (II) allgemein definiert. In dieser Formel haben X, $R^1$ und p vorzugsweise diejenigen Bedeutungen, die bereits im Zusammenhang mit der Beschreibung der erfindungsgemäßen Stoffe der Formel (I) vorzugsweise für diese Reste bzw. diesen Index genannt werden.

Die gemäß der vorliegenden Erfindung einsetzbaren Verbindungen der Formel (II) sind bekannte Verbindungen, die in der JP-OS 23267/1976 beschrieben sind.

Als Beispiele seien erwähnt:

1-(2,4-Dichlorbenzoylmethyl)-1,2,4-triazol,
1-Benzoylmethyl-1,2,4-triazol,
1-(4-Methylbenzoylmethyl)-1,2,4-triazol,
1-(4-Methoxybenzoylmethyl)-1,2,4-triazol,

1-(4-Chlorbenzoylmethyl)-1,2,4-triazol und
1-(3,4-Dichlorbenzoylmethyl)-1,2,4-triazol.

Die bei der Herstellung von Zwischenprodukten der Formel (IV) weiterhin als Ausgangsstoffe benötigten Verbindungen sind durch die Formel (III) allgemein definiert. In dieser Formel haben Y, $R^2$, g, l und m vorzugsweise diejenigen Bedeutungen, die bereits im Zusammenhang mit der Beschreibung der erfindungsgemäßen Stoffe der Formel (I) vorzugsweise für diese Reste bzw. diese Indices genannt wurden.

Die Verbindungen der Formel (III) sind bereits bekannt (vgl. Chemical Abstracts 43, 121 h (1949) und Chemical Abstracts 48, 13 963 s (1954)).

Als Beispiele für Verbindungen der Formel (III) seien genannt:

2-Phenoxyethylamin,
2-(4-Flurphenoxy)ethylamin,
2-(2,6-Xylyloxy)ethylamin,
2-(2,6-Dichlorphenoxy)ethylamin,
2-(2,4-Dichlorphenoxy)ethylamin,

2-(2,4,6-Trichlorphenoxy)ethylamin,
2-Phenylthioethylamin,
3-Phenoxypropylamin,
3-(2-Tolyloxy)propylamin,
3-(3-Tolyloxy)propylamin,
3-(4-Tolyloxy)propylamin,
3-(2-Chlorphenoxy)propylamin,
3-(3-Chlorphenoxy)propylamin,
3-(4-Chlorphenoxy)propylamin,
3-(4-Chlorphenoxy)propylamin,
3-(4-Fluorphenoxy)propylamin,
3-(4-tert-Butylphenoxy)propylamin,
3-(2-Methoxyphenoxy)propylamin,
3-(4-Methoxyphenoxy)propylamin,
3-(3-Trifluormethylphenoxy)propylamin,
3-(4-Nitrophenoxy)propylamin,
3-(2,4- Dichlorphenoxy)propylamin,
3-(3,4- Dichlorphenoxy)propylamin,
3-(3,5- Dichlorphenoxy)propylamin,
3-(2,6- Dichlorphenoxy)propylamin
3-(2,6-Xylyloxy)propylamin,
3-(4-Methoxyiminomethylphenoxy)propylamin,
4-Phenoxybutylamin,
4-(2-Methoxyphenoxy)butylamin,
4-(2,6-Xylyloxy)butylamin,
5-Phenoxypentylamin,
6-Phenoxyhexylamin und
2-Methyl-3-phenoxypropylamin.

Als Reduktionsmittel kommen bei der Durchführung des erfindungsgemäßen Verfahrens zur Herstellung der ß-substituierten Amino-phenethyl-azol-Derivate der Formel (I) vorzugsweise komplexe Metallhydride in Frage. Beispielhaft genannt seien:

Natriumborhydrid,
Aluminiumborhydrid,
Kaliumborhydrid
Lithiumborhydrid,
Lithiumaluminiumhydrid,
Natriumcyanidborhydrid,
Lithiumcyanidborhydrid,
Dimethylaminoboran-Komplex und
Diethylsilan-Chlorotris(triphenylphosphin)rhodium-Komplex.

Sowohl bei dem Verfahren zur Herstellung der erfindungsgemäßen Verbindungen der Formel (I) als auch bei dem Verfahren zur Herstellung der Zwischenprodukte der Formel (IV) können als Verdünnungsmittel alle üblichen inerten Solventien eingesetzt werden. Als bevorzugt verwendbare Löungsmittel genannt seien Wasser, aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether,

Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die Reaktionstemperaturen können sowohl bei der Durchführung des Verfahrens zur Herstellung der Stoffe der Formel (I) als auch bei dem Verfahren zur Herstellung der Zwischenprodukte der Formel (IV) innerhalb eines größeren Bereiches variiert werden. Im allgemeinen arbeitet man bei Temperaturen zwischen -20°C und dem Siedepunkt des Reaktionsgemisches, vorzugsweise zwischen 0°C und 100°C.

Bei der Durchführung der erfindungsgemäßen Umsetzungen arbeitet man im allgemeinen unter atmosphärischem Druck. Es ist jedoch auch möglich, unter erhöhtem oder vermindertem Druck zu arbeiten.

Bei dem Verfahren zur Herstellung der Zwischenprodukte der Formel (IV) setzt man auf 1 Mol einer Verbindung der Formel (II) im allgemeinen 1 bis 1,05 Mol einer Verbindung der Formel (III) ein. Die Verbindungen der Formel (IV) werden erhalten, indem man eine azeotrope

Entwässerung durchführt. Die Aufarbeitung des Reaktionsgemisches und die Isolierung der Verbindungen der Formel (IV) erfolgen nach üblichen Methoden. Die Zwischenprodukte der Formel (IV) können unmittelbar nach ihrer Herstellung in Lösung oder auch nach vorheriger Isolierung zur anschließenden Synthese der erfindungsgemäßen Verbindungen der Formel (I) eingesetzt werden.

Bei der Durchführung des Verfahrens zur Herstellung der erfindungsgemäßen Stoffe der Formel (I) setzt man 1 Mol einer Verbindung der Formel (IV) im allgemeinen mit einer äquivalenten Menge oder auch mit einem Überschuß an einem komplexen Metallhydrid um. Aufarbeitung des Reaktionsgemisches und Isolierung der Verbindungen der Formel (I) erfolgen nach üblichen Methoden.

Die erfindungsgemäßen Stoffe der Formel (I) besitzen eine starke fungizide Wirksamkeit und können zur Bekämpfung von phytopthogenen Pilzen in der Landwirtschaft und im Gartenbau eingesetzt werden.

Fungizide Mittel werden im Pflanzenschutz eingesetzt zur Bekämpfung von

Plasmodiophoromycetes,
Oomycetes,
Chytridiomycetes,
Zygomycetes,
Ascomycetes,
Basidiomycetes und
Deuteromycetes,
insbesondere zur Bekämpfung von
Graufäule (Botrytis cinerea) verschiedener Nutzpflanzen,
Pulver-Mehltau (Sphaerotheca fuliginea) der Gurke und
Braunfäule der Tomate (Phytophtora infestans).

Die gute Tolerierung der aktiven Verbindungen durch die Pflanzen bei den zur Bekämpfung der Pflanzenkrankheiten erforderlichen Konzentrationen ermöglicht die Behandlung der oberirdischen Pflanzenteile, der Materialien zur vegetativen Vermehrung und des Saatguts sowie des Bodens.

Die aktiven Verbindungen können als gebräuchliche Formulierungen zubereitet werden, etwa als Lösungen, Emulsionen, Suspensionen, Pulver, Schäume, Pasten, Granulat, Aerosol, mit der aktiven Verbindung imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die

kalte Vernebelung und die warme Vernebelung nach dem Ultra-Low-Volume-Verfahren.

Diese Formulierungen können in bekannter Weise hergestellt werden, beispielsweise durch Vermischen der aktiven Verbindungen mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel hauptsächlich geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Erdöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist auch möglich, farbgebende Mittel, etwa anorganische Pigmente wie beispielsweise Eisenoxid, Titanoxid und Preußisch Blau oder organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-% der aktiven Verbindung.

Die aktiven Verbindungen gemäß der Erfindung können in den Formulierungen oder den verschiedenen Anwendungsformen im Gemisch mit anderen bekannten aktiven Verbindungen vorliegen, etwa mit Fungiziden, Bakteriziden, Insektiziden, Akariziden, Nematiziden, Herbiziden, vogelabweisenden Mitteln, Wachstumsfaktoren, Pflanzennährstoffen und Mitteln zur Verbesserung der Bodenstruktur.

Die aktiven Verbindungen können als solche oder in Form der aus ihnen durch weitere Verdünnung hergestellten Formulierungen oder verschiedenen Anwendungsformen zur Anwendung gebracht werden, etwa als gebrauchsfertige Lösungen, Emulsionen, Suspensionen, Pulver, Pasten und Granulat. Sie werden in der üblichen Weise ausgebracht, beispielsweise durch Bewässern, Eintauchen, Sprühen, Zerstäuben, Vernebeln, Verdampfen, Injizieren, Bilden einer Aufschlämmung, Aufpinseln, Stäuben, Streuen, Trockendüngung, Feuchtdüngung, Naßdüngung, Schlammdüngung oder Überkrusten.

Bei der Behandlung von Pflanzenteilen können die Konzentrationen der aktiven Verbindungen innerhalb eines wesentlichen Bereichs variiert werden. Diese reichen allgemein von 1 bis 0,0001 Gew.-%, vorzugsweise von 0,5 bis 0,001 Gew.-%.

Bei der Saatgut-Behandlung werden allgemein Mengen der aktiven Verbindungen, bezogen auf die Saatgut-Menge, von 0,001 bis 50 g/kg, insbesondere von 0,01 bis 10 g/kg, eingesetzt.

Bei der Bodenbehandlung gelangen im allgemeinen Konzentrationen der aktiven Verbindungen am Wirkungsort von 0,00001 bis 0,1 Gew.-%, vorzugsweise von 0,0001 bis 0,02 Gew.-%, zur Anwendung.

Die folgenden Beispiele erläutern die vorliegende Erfindung im einzelnen. Es sei jedoch darauf hingewiesen, daß die vorliegende Erfindung nicht allein auf diese speziellen Beispiele beschränkt ist.

Herstellungsbeispiele:

Beispiel 1

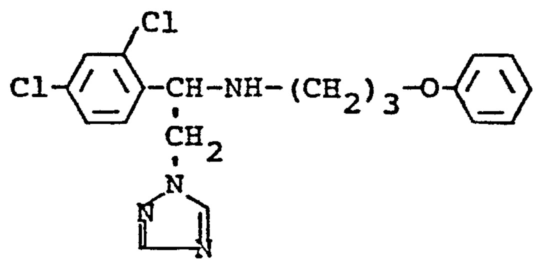

(Verbindung Nr. 1)

1-/‾ß-(3-Phenoxypropylimino)-2,4-dichlorphenethyl_7-1H-1,2,4-triazol (2 g) wurde in Methanol (30 ml) gelöst. Unter Eiskühlung wurde Natriumborhydrid (0,59 g) allmählich hinzugefügt, und die Mischung wurde 1 h bei 50°C gerührt. Nach der Reaktion wurde das Methanol abgedampft. Der Rückstand wurde mit Dichlormethan extrahiert, mit Wasser gewaschen und getrocknet. Das Lösungsmittel wurde abgedampft, wonach das gewünschte 1-/‾ß-(3-Phenoxypropylamino)-2-4-dichlorphenethyl_7-1H-1,2,4-triazol (1,6 g) erhalten wurde. (Ausbeute 80 %). $n_D^{30}$ = 1,5822.

Die in gleicher Weise wie im vorstehenden Beispiel 1 hergestellten erfindungsgemäßen Verbindungen der Formel (I) sind in Tabelle 1 aufgeführt.

0183147

Tabelle 1

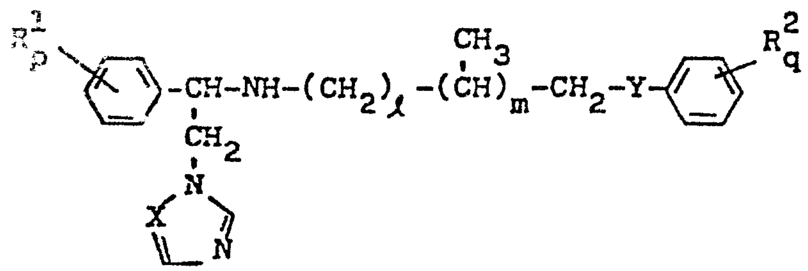

| Verbindung Nr. | $R^1_p$ | X | ℓ | m | Y | $R^2_q$ | Physikal. Konstante $n_D^{30}$ |
|---|---|---|---|---|---|---|---|
| 2 | 2,4-$Cl_2$ | N | 1 | 0 | O | - | 1.5870 |
| 3 | - | N | 1 | 0 | O | - | 1.5717 |
| 4 | 4-$CH_3$ | N | 1 | 0 | O | - | 1.5825 |
| 5 | 4-$OCH_3$ | N | 1 | 0 | O | - | 1.5805 |
| 6 | 4-Cl | N | 1 | 0 | O | - | 1.5832 |
| 7 | 3,4-$Cl_2$ | N | 1 | 0 | O | - | 1.6010 |
| 8 | 2,4-$Cl_2$ | N | 1 | 0 | O | 4-F | 1.5735 |
| 9 | 2,4-$Cl_2$ | N | 1 | 0 | O | 2,6-$(CH_3)_2$ | 1.5788 |
| 10 | 2,4-$Cl_2$ | N | 1 | 0 | O | 2,6-$Cl_2$ | 1.5934 |
| 11 | 2,4-$Cl_2$ | N | 1 | 0 | O | 2,4-$Cl_2$ | 1.5885 |
| 12 | 2,4-$Cl_2$ | N | 1 | 0 | O | 2,4,6-$Cl_3$ | 1.5960 |
| 13 | 2,4-$Cl_2$ | N | 1 | 0 | S | - | 1.5950 |
| 14 | 2,4-$Cl_2$ | N | 2 | 0 | O | 2-$CH_3$ | 1.5606 |
| 15 | 2,4-$Cl_2$ | N | 2 | 0 | O | 3-$CH_3$ | 1.5750 |
| 16 | 2,4-$Cl_2$ | N | 2 | 0 | O | 4-$CH_3$ | 1.5735 |
| 17 | 2,4-$Cl_2$ | N | 2 | 0 | O | 2-Cl | 1,5770 |
| 18 | 2,4-$Cl_2$ | N | 2 | 0 | O | 3-Cl | 1.5837 |
| 19 | 2,4-$Cl_2$ | N | 2 | 0 | O | 4-Cl | 1.5812 |
| 20 | 2,4-$Cl_2$ | N | 2 | 0 | O | 4-F | 1.5557 |

| Verbindung Nr | $R^1_p$ | X | l | m | Y | $R^2_q$ | Physikalische Konstante $n_D^{30}$ |
|---|---|---|---|---|---|---|---|
| 21 | 2,4-$Cl_2$ | N | 2 | 0 | 0 | 4-Br | 1.5941 |
| 22 | 2,4-$Cl_2$ | N | 2 | 0 | 0 | 4-$C_4H_9$-tert | 1.5598 |
| 23 | 2,4-$Cl_2$ | N | 2 | 0 | 0 | 2-$OCH_3$ | 1.5745 |
| 24 | 2,4-$Cl_2$ | N | 2 | 0 | 0 | 4-$OCH_3$ | 1.5764 |
| 25 | 2,4-$Cl_2$ | N | 2 | 0 | 0 | 3-$CF_3$ | 1.5448 |
| 26 | 2,4-$Cl_2$ | N | 2 | 0 | 0 | 4-$NO_2$ | 1.6035 |
| 27 | 2,4-$Cl_2$ | N | 2 | 0 | 0 | 2,4-$Cl_2$ | 1.5890 |
| 28 | 2,4-$Cl_2$ | N | 2 | 0 | 0 | 3,4-$Cl_2$ | 1.5900 |
| 29 | 2,4-$Cl_2$ | N | 2 | 0 | 0 | 3,5-$Cl_2$ | 1.5825 |
| 30 | 2,4-$Cl_2$ | N | 2 | 0 | 0 | 2,6-$Cl_2$ | 1.5840 |
| 31 | 2,4-$Cl_2$ | N | 2 | 0 | 0 | 2,6-$(CH_3)_2$ | 1.5675 |
| 32 | 2,4-$Cl_2$ | N | 2 | 0 | 0 | 4-CH=$NOCH_3$ | 1.5982 |
| 33 | 2,4-$Cl_2$ | N | 3 | 0 | 0 | - | 1.5780 |
| 34 | 2,4-$Cl_2$ | N | 3 | 0 | 0 | 2-$OCH_3$ | 1.5776 |
| 35 | 2,4-$Cl_2$ | N | 3 | 0 | 0 | 2,6-$(CH_3)_2$ | 1.5658 |
| 36 | 2,4-$Cl_2$ | N | 4 | 0 | 0 | - | 1.5740 |
| 37 | 2,4-$Cl_2$ | N | 5 | 0 | 0 | - | 1.5672 |
| 38 | - | N | 2 | 0 | 0 | - | 1.5708 |
| 39 | - | N | 2 | 0 | 0 | 4-$CH_3$ | 1.5667 |
| 40 | - | N | 2 | 0 | 0 | 4-Cl | 1.5732 |
| 41 | - | N | 3 | 0 | 0 | - | 1.5699 |
| 42 | - | CH | 1 | 0 | 0 | - | 1.5803 |
| 43 | - | CH | 2 | 0 | 0 | - | 1.5750 |
| 44 | 2,4-$Cl_2$ | N | 1 | 1 | 0 | - | 1.5710 |

Anmerkung: Das Zeichen "-" in den Spalten $R^1_p$ und $R^2_q$ bezeichnet die Abwesenheit eines Substituenten.

Nit 187

Beispiel 2 (Zwischenprodukte)

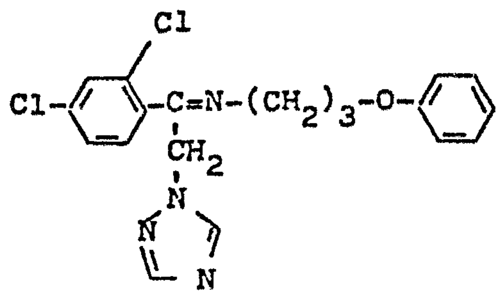

(Verbindung Nr. IV-1)

1-(2,4- Dichlorbenzoylmethyl)-1,2,4-triazol (2,56 g) und 3-Phenoxypropylamin (1,51 g) wurden in Xylol (30 ml) gelöst und 2 h einer azeotropen Entwässerungsreaktion unterworfen. Danach wurde das Lösungsmittel abgedampft, wonach 1-/¯ß-(3-Phenoxypropylimino)-2,4-dichlor-phenethyl_7-1H-1,2,4-triazol (3,65 g) erhalten wurde. (Ausbeute 94 %). $n_D^{30}$ = 1,5910.

In der gleichen Weise wie in Beispiel 2 wurden die folgenden Verbindungen und die in Tabelle 2 aufgeführten Verbindungen synthetisiert.

IV-2: 1-/¯ß-(2-Phenoxypropylimino)-2,4-dichlor-phenethyl_7-1H-1,2,4-triazol ($n_D^{30}$ = 1,5970);

IV-32: 1-/¯ß-/¯3-(4-Methoxyiminomethylphenoxy)-propylimino_7-2,4- dichlorphenethyl_7-1H-1,2,4-triazol ($n_D^{30}$ = 1,6023);

IV-33: 1-/¯ß-(4-Phenoxybutylimino)-2,4- dichlorphenethyl_7-1H-1,2,4-triazol ($n_D^{30}$ = 1,5905).

Tabelle 2

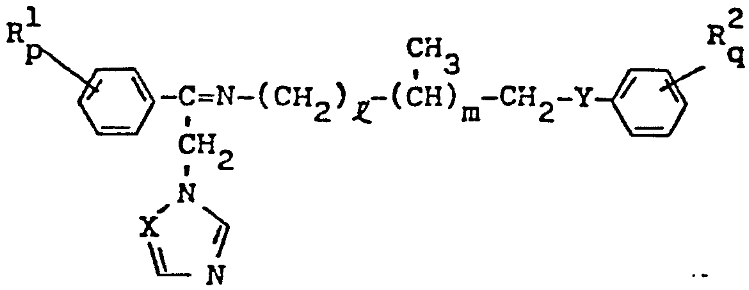

| Verbindung Nr. | $R_p^1$ | X | ℓ | m | Y | $R_q^2$ |
|---|---|---|---|---|---|---|
| IV-3 | - | N | 1 | 0 | O | - |
| IV-4 | 4-$CH_3$ | N | 1 | 0 | O | - |
| IV-5 | 4-$OCH_3$ | N | 1 | 0 | O | - |
| IV-6 | 4-Cl | N | 1 | 0 | O | - |
| IV-7 | 3,4-$Cl_2$ | N | 1 | 0 | O | - |
| IV-8 | 2,4-$Cl_2$ | N | 1 | 0 | O | 4-F |
| IV-9 | 2,4-$Cl_2$ | N | 1 | 0 | O | 2,6-$(CH_3)_2$ |
| IV-10 | 2,4-$Cl_2$ | N | 1 | 0 | O | 2,6-$Cl_2$ |
| IV-11 | 2,4-$Cl_2$ | N | 1 | 0 | O | 2,4-$Cl_2$ |
| IV-12 | 2,4-$Cl_2$ | N | 1 | 0 | O | 2,4,6-$Cl_3$ |
| IV-13 | 2,4-$Cl_2$ | N | 1 | 0 | S | - |
| IV-14 | 2,4-$Cl_2$ | N | 2 | 0 | O | 2-$CH_3$ |
| IV-15 | 2,4-$Cl_2$ | N | 2 | 0 | O | 3-$CH_3$ |
| IV-16 | 2,4-$Cl_2$ | N | 2 | 0 | O | 4-$CH_3$ |
| IV-17 | 2,4-$Cl_2$ | N | 2 | 0 | O | 2-Cl |
| IV-18 | 2,4-$Cl_2$ | N | 2 | 0 | O | 3-Cl |
| IV-19 | 2,4-$Cl_2$ | N | 2 | 0 | O | 4-Cl |
| IV-20 | 2,4-$Cl_2$ | N | 2 | 0 | O | 4-F |
| IV-21 | 2,4-$Cl_2$ | N | 2 | 0 | O | 4-Br |

Nit 187

Tabelle 2 - Fortsetzung

| Verbindung Nr. | $R^1_p$ | X | l | m | Y | $R^2_q$ |
|---|---|---|---|---|---|---|
| IV-22 | 2,4-$Cl_2$ | N | 2 | 0 | 0 | 4-$C_4H_9$-tert |
| IV-23 | 2,4-$Cl_2$ | N | 2 | 0 | 0 | 2-$OCH_3$ |
| IV-24 | 2,4-$Cl_2$ | N | 2 | 0 | 0 | 4-$OCH_3$ |
| IV-25 | 2,4-$Cl_2$ | N | 2 | 0 | 0 | 3-$CF_3$ |
| IV-26 | 2,4-$Cl_2$ | N | 2 | 0 | 0 | 4-$NO_2$ |
| IV-27 | 2,4-$Cl_2$ | N | 2 | 0 | 0 | 2,4-$Cl_2$ |
| IV-28 | 2,4-$Cl_2$ | N | 2 | 0 | 0 | 3,4-$Cl_2$ |
| IV-29 | 2,4-$Cl_2$ | N | 2 | 0 | 0 | 3,5-$Cl_2$ |
| IV-30 | 2,4-$Cl_2$ | N | 2 | 0 | 0 | 2,6-$Cl_2$ |
| IV-31 | 2,4-$Cl_2$ | N | 2 | 0 | 0 | 2,6-$(CH_3)_2$ |
| IV-34 | 2,4-$Cl_2$ | N | 3 | 0 | 0 | 2-$OCH_3$ |
| IV-35 | 2,4-$Cl_2$ | N | 3 | 0 | 0 | 2,6-$(CH_3)_2$ |
| IV-36 | 2,4-$Cl_2$ | N | 4 | 0 | 0 | - |
| IV-37 | 2,4-$Cl_2$ | N | 5 | 0 | 0 | - |
| IV-38 | - | N | 2 | 0 | 0 | - |
| IV-39 | - | N | 2 | 0 | 0 | 4-$CH_3$ |
| IV-40 | - | N | 2 | 0 | 0 | 4-Cl |
| IV-41 | - | N | 3 | 0 | 0 | - |
| IV-42 | - | CH | 1 | 0 | 0 | - |
| IV-43 | - | CH | 2 | 0 | 0 | - |
| IV-44 | 2,4-$Cl_2$ | N | 1 | 1 | 0 | - |

Anmerkung: Das Zeichen "-" in den Spalten $R^1_p$ und $R^2_q$ bezeichnet die Abwesenheit eines Substituenten.

Nit 187

Verwendungsbeispiele:

Vergleichs-Verbindung A-1:

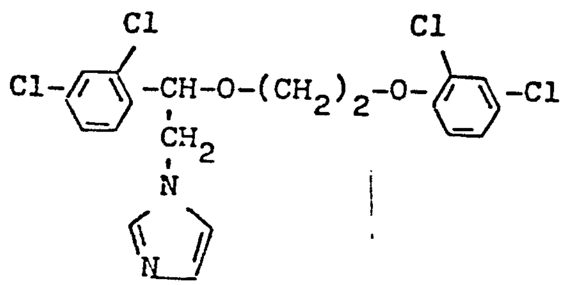

(die in der JP-OS 49369/1981 beschriebene Verbindung)

Beispiel 3

Test der Bekämpfungswirkung gegen Graufäule (Botrytis cinerea) auf Bohnen:

Herstellung einer Test-Verbindung:

| | |
|---|---|
| Test-Verbindung: | 30 Teile; |
| Organisches Lösungsmittel (Xylol): | 55 Teile; |
| Emulgator: | |
| Polyoxyethylenalkylphenylether: | 8 Teile und |
| Calciumalkylbenzolsulfonat: | 7 Teile. |

Zur Herstellung der Test-Chemikalie wurde eine aus den vorstehenden Bestandteilen bestehende Emulsion mit Wasser auf eine vorher festgelegte Konzentration verdünnt.

Test-Verfahren

Die Test-Verbindung in Form einer Emulsion wurde mit einer Sprühpistole auf die ersten Blätter von in unglasierten 9 cm-Töpfen gezogenen Vitsbohnen (Varietät:

Shin-edigawa) aufgesprüht. Einen Tag nach dem Sprühen wurde eine den Fungus tragende Agarscheibe, die durch Ausstanzen der Spitzen-Teile von Kolonien der Graufäule (Botrytis cinerea), die in einem PDA-Medium bei 20°C zwei Tage im voraus kultiviert worden waren, mittels eines Korkbohrers mit einem Durchmesser von 5 mm erhalten worden war, auf das Zentrum jedes Blattes gelegt. Die Töpfe wurden dann in einem feuchten Raum bei 20°C gehalten. Zwei Tage nach der Inokulation wurde der Durchmesser einer Läsion gemessen, und die Bekämpfungswirkung wurde berechnet.

$$\text{Bekämpfungs-Index (\%)} = \frac{\text{Durchmesser der Läsion der unbehandelten Fläche} - \text{Durchmesser der Läsion der behandelten Fläche}}{\text{Durchmesser der Läsion der unbehandelten Fläche}} \times 100$$

Die Verbindungen der Erfindung zeigten eine hervorragende Bekämpfungswirkung bei einer Konzentration von 500 ppm oder weniger (der Konzentration der aktiven Verbindung). Typische Beispiele für die Ergebnisse sind nachstehend aufgeführt.

Tabelle 3

| Verbindung Nr. | Konzentration der aktiven Komponente (ppm) | Bekämpfungs-Index (%) |
|---|---|---|
| 1 | 500 | 100 |
| 2 | 500 | 100 |
| 13 | 500 | 100 |
| 15 | 500 | 100 |
| 16 | 500 | 100 |
| 18 | 500 | 100 |
| 33 | 500 | 100 |
| A-1 (Vergleichs-Verbindung) | 1000 | 0 |

Beispiel 4

Test auf Wirksamkeit gegen den Pulver-Mehltau der Gurke:

Die wie in Beispiel 3 hergestellte Test-Verbindung in Form einer Emulsion wurde mit einer Sprühpistole auf in unglasierten 9 cm-Töpfen gezogene Gurken (Varietät: "Tokiwa", am Boden kriechend) im 2-Blatt-Stadium aufgesprüht. Einen Tag nach dem Sprühen wurde eine Sporen-Suspension des Pulver-Mehltau-Pathogens (Sphaerotheca fuliginea) durch Sprühen inokuliert. Die Töpfe wurden in einem Raum mit einer konstanten Temperatur von 23°C stehen gelassen, und zehn Tage später wurde der Grad der Erkrankung aufgrund des nachfolgenden Bewertungsmaßstabs durch das prozentuale Verhältnis der Läsionen zeigenden Flächen bestimmt, und der Bekämpfungsindex wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 15 |
| 3 | 16 bis 30 |
| 4 | 31 bis 50 |
| 5 | 51 oder mehr |

$$\text{Bekämpfungs-Index (\%)} = \frac{\text{Grad der Erkrankung der unbehandelten Fläche} - \text{Grad der Erkrankung der behandelten Fläche}}{\text{Grad der Erkrankung der unbehandelten Fläche}} \times 100$$

Die Verbindungen der Erfindung zeigten eine hervorragende Bekämpfungswirkung bei einer Konzentration von 50 ppm oder weniger (der Konzentration der aktiven Verbindung). Typische Beispiele sind nachstehend aufgeführt.

Tabelle 4

| Verbindung Nr. | Konzentration der aktiven Komponente (ppm) | Bekämpfungs-Index (%) |
|---|---|---|
| 1 | 50 | 100 |
| 2 | 50 | 100 |
| 6 | 50 | 100 |
| 8 | 50 | 100 |
| 13 | 50 | 100 |
| 33 | 50 | 100 |
| 36 | 50 | 100 |
| A-1 (Vergleichs-Verbindung) | 50 | 0 |

Patentansprüche

1. ß-Substituierte Aminophenethylazol-Derivate der Formel (I)

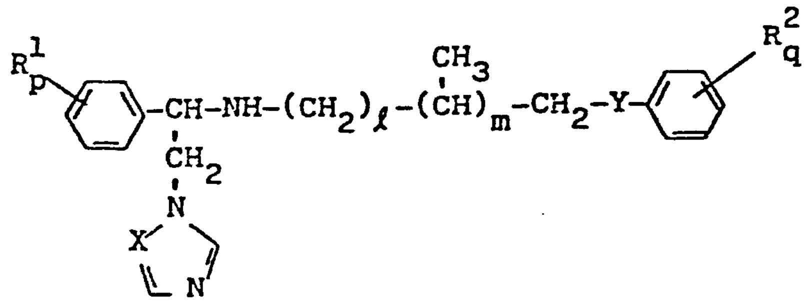

(I)

,

in der

X N oder CH bezeichnet,

Y ein Sauerstoff- oder Schwefel-Atom bezeichnet,

$R^1$ eine Niederalkyl-Gruppe, ein Halogen-Atom oder eine Niederalkoxy-Gruppe bezeichnet,

$R^2$ eine Niederalkyl-Gruppe, ein Halogen-Atom, eine Niederalkoxy-Gruppe, eine Trifluor-methyl-Gruppe, eine Nitro-Gruppe oder eine Methoxyiminomethyl-Gruppe bezeichnet,

p 0, 1 oder 2 bezeichnet,

q 0, 1, 2 oder 3 bezeichnet,

$\ell$ 1, 2, 3, 4 oder 5 bezeichnet und

m 0 oder 1 bezeichnet.

2. Verbindungen gemäß Anspruch 1, dadurch gekennzeichnet, daß

X für Stickstoff steht,

Y für Sauerstoff oder Schwefel steht,

$R^1$ für Alkyl mit 1 bis 4 Kohlenstoffatomen, Fluor, Chlor, Brom, Iod oder Alkoxy mit 1 bis 4 Kohlenstoffatomen steht,

$R^2$ für Alkyl mit 1 bis 4 Kohlenstoffatomen, Fluor, Chlor, Brom, Iod, Alkoxy mit 1 bis 4 Kohlenstoffatomen, Trifluormethyl, Nitro oder Methoximinomethyl steht,

p für 0, 1 oder 2 steht,

q für 0, 1, 2 oder 3 steht,

l für 1, 2 oder 3 steht,

m für 0 oder 1 steht.

3. Verbindungen nach Anspruch 1, ausgewählt aus 1-/ß-(2-Phenoxyethylamino)-2,4-dichlorphenethyl/-1H-1,2,4-triazol der Formel

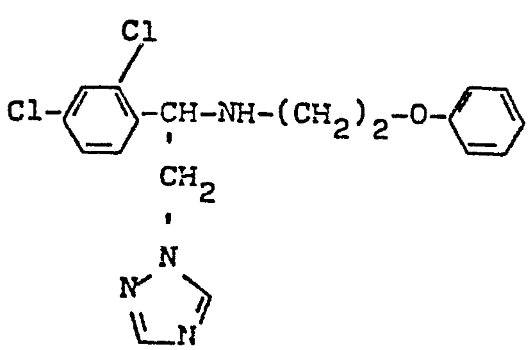

,

1-/β-(3-Phenoxypropylamino)-2,4-dichlorphenethyl/-1H-1,2,4-triazol der Formel

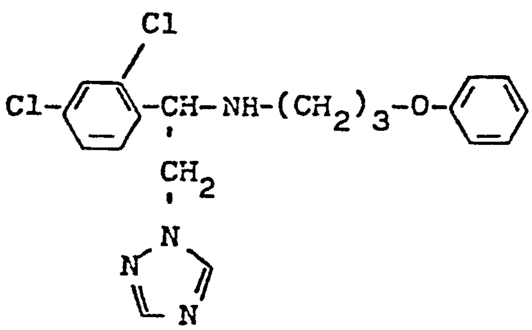

,

1-/β-/3-(4-chlorphenoxy)-propylamino/-2,4-dichlorphenethyl/-1H-1,2,4-triazol der Formel

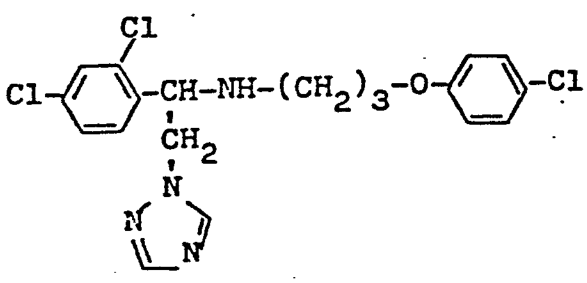

,

1-/β-/3-(4-tolyloxy)propylamino/-2,4-dichlorphenethyl/-1H-1,2,4-triazol der Formel

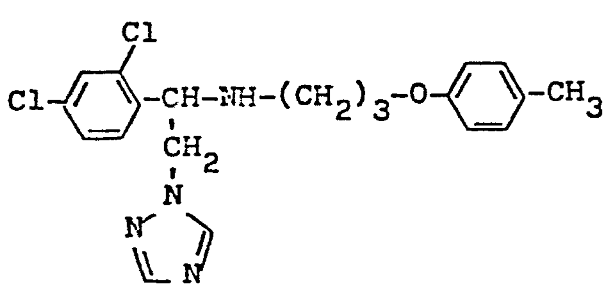

und

Nit 187

1-/-ß-(2-Methyl-3-phenoxypropylamino)-2,4-dichlor-phenethyl7-1H-1,2,4-triazol der Formel

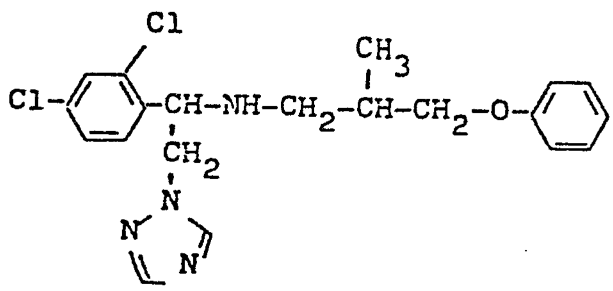

4. Verfahren zur Herstellung von ß-substituierten Aminophenethylazol-Derivaten der Formel (I)

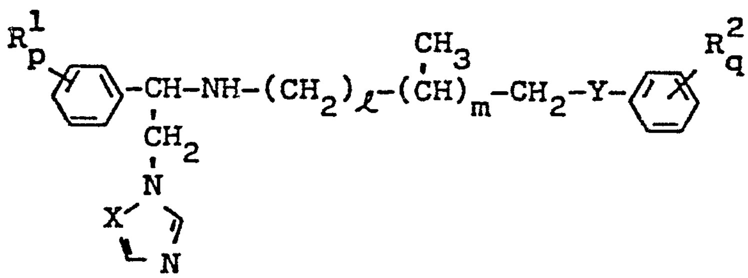 (I)

,

in der

X N oder CH bezeichnet,

Y ein Sauerstoff- oder Schwefel-Atom bezeichnet,

$R^1$ eine Niederalkyl-Gruppe, ein Halogen-Atom oder eine Niederalkoxy-Gruppe bezeichnet,

$R^2$ eine Niederalkyl-Gruppe, ein Halogen-Atom, eine Niederalkoxy-Gruppe, eine Trifluormethyl-Gruppe, eine Nitro-Gruppe oder eine Methoxyiminomethyl-Gruppe bezeichnet,

p 0, 1 oder 2 bezeichnet,
q 0, 1, 2 oder 3 bezeichnet,
l 1,2,3,4 oder 5 bezeichnet und
m 0 oder 1 bezeichnet,

dadurch gekennzeichnet, daß man Verbindungen der Formel

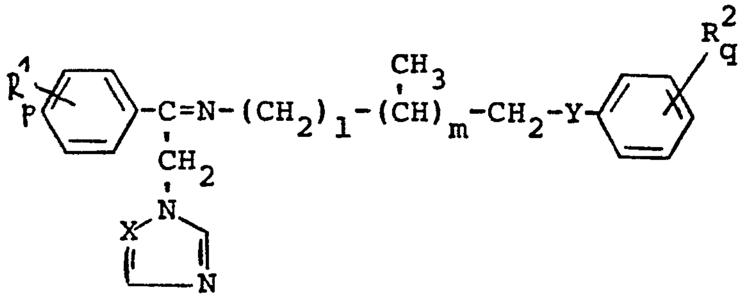

(IV)

in welcher

X, Y, $R^1$, $R^2$, p, q, l und m die oben angegebene Bedeutung haben,

gegebenenfalls in Gegenwart eines inerten Verdünnungsmittels reduziert.

5. Fungizide Mittel für Landwirtschaft und Gartenbau, dadurch gekennzeichnet, daß sie wenigstens ein ß-substituiertes Aminophenethylazol-Derivat der Formel (I) nach Anspruch 1 enthalten.

6. Verfahren zur Bekämpfung von phytopathogenen Pilzen, dadurch gekennzeichnet, daß ß-substituierte Aminophenethylazol-Derivate der Formel (I) nach Anspruch 1 auf Pflanzen und/oder deren Lebensraum ausgebracht werden.

7. Verwendung von ß-substituierten Aminophenethylazol-Derivaten der Formel (I) nach Anspruch 1 zur Bekämpfung von phytopathogenen Pilzen.

8. Verfahren zur Herstellung fungizider Mittel für Landwirtschaft und Gartenbau, dadurch gekennzeichnet, daß ß-substituierte Aminophenethylazol-Derivate der Formel (I) nach Anspruch 1 mit Streckmitteln und/oder grenzflächenaktiven Stoffen vermischt werden.

9. Verbindungen der Formel (IV)

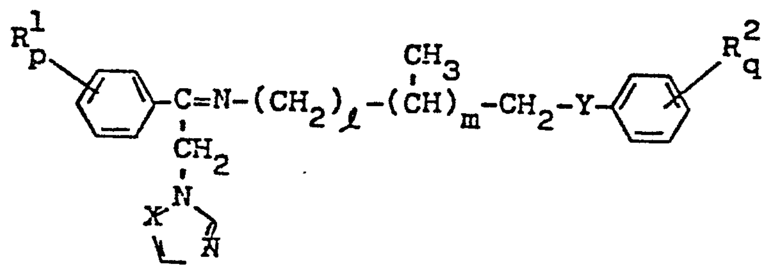

(IV)

in der

X N oder CH bezeichnet,
Y ein Sauerstoff- oder Schwefel-Atom bezeichnet,
$R^1$ eine Niederalkyl-Gruppe, ein Halogen-Atom oder eine Niederalkoxy-Gruppe bezeichnet,
$R^2$ eine Niederalkyl-Gruppe, ein Halogen-Atom, eine Niederalkoxy-Gruppe, eine Trifluormethyl-Gruppe, eine Nitro-Gruppe oder eine Methoxyiminomethyl-Gruppe bezeichnet,
p 0, 1 oder 2 bezeichnet,
q 0, 1, 2 oder 3 bezeichnet,
$\ell$ 1, 2, 3, 4 oder 5 bezeichnet und
m 0 oder 1 bezeichnet.

10. Verfahren zur Herstellung von Verbindungen der Formel (IV)

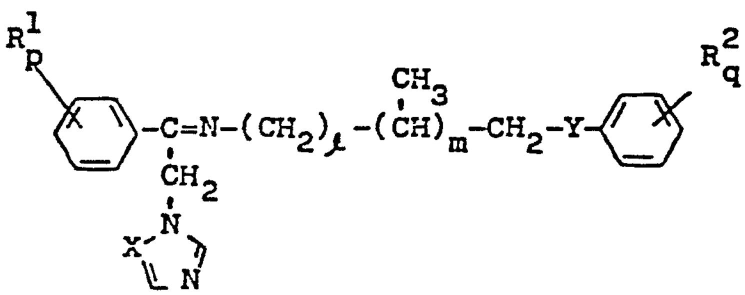

(IV)

,

in der
X N oder CH bezeichnet,
Y ein Sauerstoff- oder Schwefel-Atom bezeichnet,
$R^1$ eine Niederalkyl-Gruppe, ein Halogen-Atom oder eine Niederalkoxy-Gruppe bezeichnet,
$R^2$ eine Niederalkyl-Gruppe, ein Halogen-Atom, eine Niederalkoxy-Gruppe, eine Trifluormethyl-Gruppe, eine Nitro-Gruppe oder eine Methoxyiminomethyl-Gruppe bezeichnet,
p 0, 1 oder 2 bezeichnet,
q 0, 1, 2 oder 3 bezeichnet,
$\ell$ 1, 2, 3, 4 oder 5 bezeichnet und
m 0 oder 1 bezeichnet,

dadurch gekennzeichnet, daß Verbindungen der Formel (II)

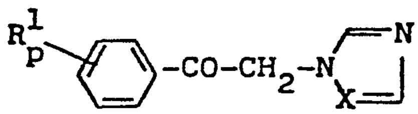

(II)

,

in der X, $R^1$ und p die oben angegebenen Bedeutungen haben,

mit Verbindungen der Formel (III)

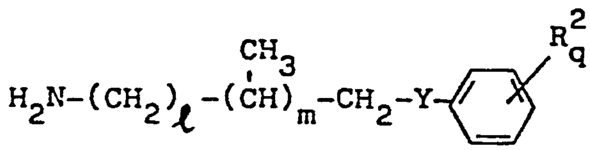

(III)

,

in der Y, $R^2$, q, $\ell$ und m die angegebenen Bedeutungen haben,

gegebenenfalls in Gegenwart eines inerten Verdünnungsmittels umgesetzt werden.